# EUROPEAN PATENT SPECIFICATION

(11) **EP 1 423 102 B1**
(45) Date of publication and mention of the grant of the patent: **21.11.2007**
(21) Application number: 02797674.5
(22) Date of filing: 04.09.2002
(51) Int. Cl.: A61K 9/70, A61P 31/10

(54) **PLASTER FOR THE TREATMENT OF DYSFUNCTIONS AND DISORDERS OF NAIL GROWTH**
PFLASTER ZUR BEHANDLUNG VON FUNKTIONSSTÖRUNGEN UND STÖRUNGEN DES NAGELWACHSTUMS
PANSEMENT POUR LE TRAITEMENT DE DISFONCTIONNEMENTS ET DE TROUBLES RELATIFS A LA POUSSE DES ONGLES

(30) Priority: 04.09.2001 EP 01121202
(43) Date of publication of application: 02.06.2004
(73) Proprietor: Trommsdorff GmbH & Co.KG Arzneimittel, 52475 Alsdorf (DE)
(72) Inventor: SUSILO, Rudy, 50999 Köln (DE)
(74) Representative: Arth, Hans-Lothar
(86) International application number: PCT/EP2002/009912
(87) International publication number: WO 2003/020250

(56) References cited:
- WO-A-94/08591
- WO-A-99/40955
- US-A- 5 181 914
- US-A- 5 464 610
- US-A- 5 696 164
- US-A- 5 753 256
- US-A- 5 861 142
- US-B1- 6 303 140

## Description

The present invention relates to plasters for prophylaxis and/or treatment of a dysfunctions or disorders of nail growth, the use of said plasters and methods for prophylaxis and/or treatment of a dysfunction or disorder of nails and/or nail growth using said plasters.

### Background of the invention

Plasters for prophylaxis and/or treatment of a dysfunction or disorder of nail growth are not described in the literature while plasters for the treatment of, for example, onychomycosis are known. For instance, WO-A-99/40955 discloses a pressure sensitive adhesive matrix patch for the treatment of onychomycosis. This device for treating fungal infections of toenails and fingernails is made up of an occlusive backing layer and a pressure-sensitive adhesive matrix layer wherein an effective amount of an antifungal agent is uniformly dispersed, optionally with a chemical enhancer. The matrix layer has a first surface adhering to the backing layer and a second surface adapted to be in diffusional contact with the infected nail and surrounding skin area.

A method for treating onychomycosis is described in US-A-5 464 610. Within said method a plaster preparation is used comprising salicylic acid or a salt, ester or mixture thereof. Said plaster preparation is attached to a carrier and the salicylic acid is present in the plaster preparation in an amount ranging from 10 to 80% by weight of the preparation.

Nail evulsion compositions and methods for evulsing nails and treating nail and nail bed infections are disclosed in US-A-5 993 790. Claimed is a topical nail enamel composition comprising water-based nail lacquer, a preservative, urea, and a natural additive. Said nail enamel composition is suitable for the treatment of fungal, yeast, and bacterial infections of the nails and the nail beds.

US-A-5 181 914 discloses an adhesive gel pad for treating onychomycosis comprising an occlusive layer, a permeation enhancer, and an antimycotic agent.

US-A-5 696 164 discloses a bandage for the topical administration of medication to the nail for the treatment of onychomycosis comprising a backing, an antimycotic, a penetration enhancer and adhesive and additives.

WO 94 08591 A discloses a method of treating onychomycosis by applying a plaster comprising the antimycotic salicylic acid, an adhesive and additives.

WO 98 16251 A discloses a carrier system for the permeation of active compounds into nails or skin. The active compounds can be chosen from antimycotics, antibiotics, antiseptics and corticosteroids.

EP 0913154 A discloses a nail varnish for the treatment of psoriasis of the nails comprising a corticosteroid, salicylic acid and additives.

However none of the above documents suggest the use of an antimycotic agent for prophylaxis and/or treatment of dysfunctions or disorders of nails, nail structure and/or nail growth which is not due to onychomycosis.

US-A-5 753 256 discloses a plaster for the treatment of nail mycoses which consists of a flexible covering film, a layer of an acrylate polymer matrix, inseparably linked to said covering film, and comprises an active compound selected form miconazole, econazole, isoconazole, tioconazole, terconazole, oxiconazole, ketoconazole, itraconazole, tolciclate, sulbentine, haloprogin, griseofulvin, cyclopirox, terbinafin, and salts of these compounds.

It is object of the present invention to provide a plaster for prophylaxis and/or treatment of dysfunctions or disorders of nails, nail structure and/or nail growth which is not due to onychomycosis.

This object is solved by the plaster of the independent claims and the use of said plaster. Further advantageous features, aspects and details of the invention are evident from the dependent claims, the description, and the examples of the present application.

### Description of the invention

The present application relates to plasters for prophylaxis and/or treatment of dysfunctions or disorders of nails and/or nail growth. Said plasters comprise a layer being designed to be in close contact with the nail and optionally with the surrounding skin.

Said layer comprises:
a) an adhesive;
b) at least one skin and/or nail permeation enhancer;
c) at least one therapeutically effective amount of an antimycotic agent contained in said contact layer; and
d) at least one additive selected from the group comprising binders, cross linkers, softeners, solvents, fillers, and/or antioxidants.

Plasters constructed of two or more layers are also useful for the purposes disclosed herein. A preferred embodiment comprises a layer which is separated into a backing layer and a contact layer wherein the contact layer is attached to said backing layer and is designed to be in close contact with the nail and optionally with the surrounding skin. Said contact layer comprises:
a) an adhesive;
b) at least one skin and/or nail permeation enhancer;
c) at least one therapeutically effective amount of an antimycotic agent contained in said contact layer; and
d) at least one additive selected from the group comprising binders, cross linkers, softeners, solvents, fillers, and/or antioxidants.

The flexible backing layer holds and presses the plaster against the nail and skin in order to increase migration from the contact layer into the nail, nail beds, and surrounding skin. Furthermore, the backing layer protects the contact layer form being contaminated. A preferred embodiment of said plaster comprises a colorless backing layer or a backing layer having an amber-like color. Another preferred embodiment comprises a flexible and/or occlusive backing layer. The plaster has sufficient flexibility in order to seal the affected nail exactly even if the nail has an uneven surface.

Another preferred embodiment of the present invention comprises a contact layer which is further divided into a central layer and a drug containing layer. The central layer is designed to be in close contact with the backing layer and the drug containing layer and contains no pharmaceutically active agent while the drug containing layer comprises a therapeutically effective amount of an antimycotic agent as summarized below.

The backing layer is preferably made of polyethylene (such as LDPE, Plastotrans^{®}), polypropylene, polyurethane, polyester (such as Revatrans^{®}, TRICON GmbH, Freiburg), Guttagena^{®} PVC NBR foil (such as Guttagena^{®} WK 68, Kalle Pentaplast, Germany), cotton, cotton/viscose, silk, polyethylenterephthalate (such as Hostaphan^{®} RN 36 sil; Hostaphan^{®} RN 100 sil, Loparex, Apeldoorn, The Netherlands), ethylene-methacrylic acid copolymers and/or mixtures of these materials. More preferably are siliconized polymers and/or copolymers.

As used herein, the term "contact layer" refers to a biocompatible adhesive containing said antimycotic agent and optionally further ingredients and/or additives with special biological functions suitable to allow and support migration and penetration of the antimycotic agent into the nails, nail beds, and the associated skin. The contact layer is inseparably linked with the backing layer, preferably with a flexible occlusive backing layer.

A sufficiently large amount of said antimycotic agent can be embedded into said adhesive which is preferably a gel-like or rubber-like adhesive in order to admit and maintain a continuing flow of the antimycotic agent through the skin and nail for a longer time, preferably for one week.

The inventive plaster can be manufactured in any suitable shape, such as round, oval, rectangular or quadratic shape. Preferred plaster sizes are 0.5 cm², 0.85 cm², 1.5 cm², 2.3 cm², 2.5 cm², and 4.0 cm².

Suitable therapeutically effective amounts of said antimycotic agents are 0.005 - 10 mg per cm² plaster, preferably 0.01 - 5 mg per cm² plaster, more preferably 1 - 4 mg, and most preferably 1 - 2 mg per cm² plaster.

These antifungal agents can roughly by divided into five groups comprising polyenes such as amphotericin B and nystatin; azoles especially imidazoles such as miconazole and sertaconazole; triazoles such as itraconazole, fluconazole, and voriconazole; allylamines such as naftifidine and terbinafine; morpholines such as amorolfine; and benzofuranes such as griseofulvin.

The inventive plaster contains a pharmaceutically active amount of at least one antimycotic agent, such as fluconazole (Diflucan^{®}), butoconazole, enilconazole, fenticonazole, sulconazole, naftifidine, clioquinol, iodoquinol, rimoprogin, griseofulvin, terbinafine (Lamisil^{®}, Novartis Pharma), clotrimazole, itraconazole (Sempera^{®}, Janssen Pharmaceutical), tioconazole, miconazole, tolnaftate, pyrogallol, econazole, isoconazole, terconazole, oxiconazole, voriconazole, amphotericin B, nystatin, tolciclate, sulbentine, ketoconazole, ciclopirox (Batrafen^{®}, Aventis Pharma), amorolfine, bifonazole, sodium pyrithione, salicylic acid and/or salts of these agents.

The plasters and plaster preparations of the state of the art use these antimycotic agents for the treatment of fungal infections of the nails, i.e. onychomycosis. The present invention reveals now for the first time the use of said pharmaceutically active agents for prophylaxis and/or treatment of dysfunctions or disorders of nails and/or nail growth and destruction of nail structure which are not onychomycosis. Especially, the prophylaxis and/or treatment of onychodystrophy which is not caused by onychomycosis is disclosed.

Onychomycosis, as a fungal infection, is regarded as a subgroup of onychodystrophy. Thus, infections caused by fungi are excluded from the scope of this invention.

Onychodystrophy comprises a number of nail dysfunctions and disorders such as onychocryptosis, melanonychia striata, white line disease, chronic paronychia, discolored nails, thickened nails, Unguis inflexus, coilonychia, scleronychia, onychogryphosis, onychauxis, onychoschisis, onychorrhexis, trachyonychia, cleaved and split nails.

Onychodystrophy can be caused or induced by fungal infections (onychomycosis) which is excluded from the scope of this invention.

The most prominent group of onychodystrophy apart from onychomycosis are induced by diseases of the skin such as neurodermitis (atopic eczema), and psoriasis. Furthermore, bacterial or viral infections are capable of causing or inducing onychodystrophy.

Also drugs such as antibiotics, anticoagulative agents, ACE inhibitors, betablockers, thiazides, cytostatic agents and the like are known to cause onychodystrophy. Another reason for onychodystrophy are systemic diseases such as avitaminoses, kidney failure, and heart failure. Another reason for onychodystrophy is the contact with chemical compounds such as acids, bases, oxidants and the like which cause bums, cauterizations, and also physical influences resulting in mechanical destruction of the nail plate. Finally, idiopathic causes exist for dysfunctions and/or disorders of the nail.

As used herein, the term "nail" refers to fingernails and toenails of mammals, especially humans.

A preferred embodiment of said plaster comprises a contact layer designed in such a way that said contact layer seals the affected nail almost perfectly which results in an almost quantitative exclusion of air. Furthermore, the inventive plaster may optionally contain an additional oxygen scavenger. Suitable oxygen scavenger comprise transition metal chelates or complexes with, for instance salicylic acid and/or salicylate and/or polycarboxylic acids, or oxidizable organic acids or alcohols in combination with a catalyzing agent.

Suitable skin and/or nail permeation enhancer are well known to a person skilled in the art and may be selected from the group comprising of fatty acids, fatty acid esters, fatty acid amides, fatty alcohols, 2-(2-ethoxyethoxy)-ethanol, esters of glycerol, glycerol monolaurate, propylene glycol, polyethylene glycols, unsaturated polyglycolized glycerides (Labrafil M1944CS^{®}, Gattefosse), saturated polyglycerides (Labrasol^{®}, Gattefosse), a partial glyceride of ricinoleic acid (Softigen^{®}, Hüls), Labrafac Hydro WL1219^{®} (Gattefosse), Estasan^{®} (Gattefosse), α-hydroxy acids, dimethylsulfoxide, decylmethylsulfoxide, pyrrolidones, salicylic acid, lactic acid, myristol, isopropyl myristate, dimethylformamide, dimethylacetamide, sodium dodecylsulfate, phospholipides, Transcutol^{®} (Gattefosse), Eutanol^{®} (Henkel), as well as mixtures comprising oleic acid / 2-(2-ethoxyethoxy)-ethanol, oleic acid / Labrafil^{®}, and oleic acid / Labrafac^{®} (Gattefosse), preferably in a ratio of approximately 1:1, and the like. Also enzyme components, such as proteolytic enzymes which facilitate permeation of chemical substances through the hardened nail or keratin tissue, can be used as permeation enhancer.

Examples for most common fatty acids are capric-, lauric-, myristic-, palmitic-, margaric-, stearic-, arachidic-, behenic-, lignoceric-, myristoleic-, palmitoleic-, petroselinic-, oleic-, vaccenic-, gadoleic-, gondoic-, urucic-, nervonic-, linoleic-, γ-linolenic-, dihomo-γ-linolenic-, arachidonic-, 7,10,13,16-docosatetraenoic-, 4,7,10,13,16-docosapentaenoic-, α-linolenic-, stearidonic-, 8,11,14,17-eicosatetraenoic-, 5,8,11,14,17-eicosapentaenoic-, 7,10,13,16,19-docosapentaenoic-, 4,7,10,13,16,19-docosahexaenoic-, 5,8,11-eicosatrienoic-, tariric-, santalbic-, stearolic-, 6,9-octadecenynoic-, pyrulic-, crepenynic-, heisteric-, t8,t10-octadecadiene-12-ynoic-, 5,8,11,14-eicosatetraynoic-, cerebronic-, hydroxynervonic-, brassylic-, and thapsic acid. Also useful are the lower alkyl ester and amides of said fatty acids or the corresponding alcohols thereof. The glycerol esters may also contain one or more of said fatty acids.

The skin and/or nail permeation enhancer supports and increases the penetration and permeation of said antimycotic agent through the skin and into the nails and nail beds. The term "penetration enhancement" or "permeation enhancement" relates to an increase in the permeability of a biological membrane or skin and nails. Skin and/or nail permeation enhancer are mostly used for increasing the rate at which a pharmaceutically active ingredient permeates through said membrane. The effect of permeation enhancement can be determined by the use of a diffusion cell apparatus as described by Merrit et al. (Diffusion Apparatus for Skin Penetration, J. Controlled Release, 1984, 1, 161-162) measuring the rate of diffusion of a pharmaceutically active agent through animal or human skin.

An effective amount of a skin and/or nail permeation enhancer means an amount sufficient to provide the desired increase in membrane permeability and, accordingly, to obtain the desired depth of penetration and penetration of a sufficient amount of said antimycotic agent.

The inventive plaster preferably contains said skin and/or nail permeation enhancer in the contact layer in an amount of between 0.1% to 30% by weight of the adhesive, preferably 0.1% to 15% by weight of the adhesive, more preferably 0.5% to 10%, and most preferably 0.7% to 6% by weight of the contact layer.

The inventive plaster comprises optionally further additives selected from the group comprising binders, cross linkers, softeners, solvents, fillers, and/or antioxidants.

Said additive or said additives, if present, are contained in the layer in an amount of between 2% to 80% by weight of the contact layer, preferably 5% to 40% by weight of the contact layer, more preferably between 8% to 30%, even more preferably between 12% to 25%, and most preferably in an amount between 15% to 20% by weight of the contact layer.

Binders characterize substances that bind or "glue" powders together and make them cohesive by forming the adhesive layer, thus serving as a further "adhesive" in the formulation. Suitable binders include non-natural sugars, natural sugars such as sucrose, starches derived from wheat, corn rice and potato; synthetic and natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate, polyethylene glycol and waxes.

If present, the amount of binder in the adhesive can range from about 1 to 50% by weight of the adhesive, preferably from about 10 to about 50% by weight of the adhesive, more preferably from about 20 to about 50% by weight, even more preferably from about 30 to about 40% by weight.

Cross linkers may be selected from the group comprising cross linking agents such as aluminum acetylacetonate, acrylate-vinylacetate copolymer, aluminum acetonate, titanium acetylacetonate, titanium acetonate, and succinic acid.

If present, the amount of cross linkers in the adhesive can range from about 0.01 to 30% by weight of the adhesive, preferably from about 0.1 to about 50% by weight of the adhesive, more preferably from about 10 to about 50% by weight, even more preferably from about 30 to about 40% by weight.

Softener may be chosen from the group comprising dibutylsebacate (DBS), Macrogol^{®} (Clariant, Frankfurt, Germany) and the like.

If present, the amount of softener in the adhesive can range from about 0.001 to 25% by weight of the adhesive, preferably from about 0.01 to about 10% by weight of the adhesive, more preferably from about 0.1 to about 6% by weight, even more preferably from about 0.5 to about 3% by weight.

Suitable solvents for the inventive plaster may be selected form purified water; ketones such as acetone, butanone, 2-pentanone, 3-pentanone; alcohols such as ethanol, propanol, isopropanol, butanol, isobutanol, sec.-butanol, tert.-butanol; esters such as acetic acid ethyl ester, acetic acid propyl ester and the like. Furthermore, mixtures of said solvents can also be used. Suitable co-solvents may be used together with the above-mentioned solvents or mixtures of solvents, said co-solvents may be selected from the group comprising lactic acid, salicylic acid, succinic acid, urea, Miglyol^{®} 812 (Chemische Werke Hüls, Marl, Germany), triglycerides, ethyloleate, glycerylmonododecanoate, olein, oleate, Macrogol^{®} 6000, and lecithin.

If present, the amount of solvents or the total amount of solvents and co-solvents in the adhesive can range from about 0.5 to 70% by weight of the adhesive, preferably from about 3 to about 60% by weight of the adhesive, more preferably from about 10 to about 50% by weight, even more preferably from about 20 to about 40% by weight, and most preferably from about 10 to about 30% by weight of the adhesive.

Fillers may be chosen from the group comprising silica, silicic acid, preferably colloidal silica and colloidal silicic acid, lactose, Aerosil^{®} such as Aerosil^{®} 200 (Degussa-Hüls, Frankfurt, Germany), starch, Bentonit^{®} (Südchemie, Mannheim, Germany) and the like. If present, the amount of fillers in the adhesive can range from about 0.01 to 15% by weight of the adhesive, preferably from about 0.1 to about 10% by weight of the adhesive, more preferably from about 0.3 to about 6% by weight, even more preferably from about 0.5 to about 3% by weight.

Butylhydroxytoluene (BHT) may be mentioned as an example for a suitable antioxidant. Antioxidants are well known to a person skilled in the art and may be selected form the antioxidants of the state of the art.

If present, the amount of antioxidants in the adhesive can range from about 0.001 to 10% by weight of the adhesive, preferably from about 0.005 to about 6% by weight of the adhesive, more preferably from about 0.01 to about 3% by weight, even more preferably from about 0.05 to about 1% by weight.

Suitable adhesives for the inventive plaster may comprise acrylic copolymers, also known as "acrylic adhesives", like National Starch Durotak^{®} 80-1196, National Starch Durotak^{®} 387-2825, or Monsanto Gelva 737; polyacrylamide; rubber-based adhesives, also called "rubber adhesives", such as polyisobutylene (PIB) (e.g. Adhesive Research MA-24), polyisoprene, styrene-isoprene copolymers, or urethane rubbers; and silicone based adhesives, so called "silicone adhesives", such as Dow Bio-PSA.

The adhesives that may be used according to the invention represent a polymer, preferably an acrylate copolymer. Suitable monomers or mixtures of monomers for the manufacture of said acrylate polymer comprise methyl acrylate, methyl methacrylate, butyl acrylate, butyl methacrylate, isooctyl acrylate, isooctyl methacrylate, aminoalkyl acrylate, aminoalkyl methacrylate, aminoalkyl methacrylate copolymers (such as EUDRAGIT^{®} E 100, EUDRAGIT^{®} RL, EUDRAGIT^{®} RS, EUDRAGIT^{®} NE 30 D. commercially available from Röhm, Degussa-Hüls Group), hydroxyethyl acrylate, hydroxyethyl methacrylate, 2-ethylhexyl acrylate, 2-ethylhexyl methacrylate, acrylic acid, methacrylic acid, vinyl acetale, and glycidyl methacrylate. Acrylate-based adhesives are commercially available from National Starch Chemical B. V., Zutphen, NL, under the name Durotak^{®}. Examples of said product class are Durotak^{®} 280-2287 (51 % solution or solid matter), Durotak^{®} 326-1753 (37% solution or solid matter), Durotak^{®} 280-1753 (33% solution or solid matter), Durotak^{®} 901-1052 (48% solution or solid matter), Durotak^{®} 80-1196 (solid matter), and Durotak^{®} 387-2825 (50% solution).

The adhesive is contained in the plaster of the present invention in an amount of between 40% to 95% by weight of the plaster, preferably between 60 to 90%, more preferably between 70% to 90%, and most preferably between 80% to 90% by weight of the plaster.

The present invention discloses a combination therapy wherein the plaster is used in combination with a systemic treatment of onychomycosis or other systemic treatments for dysfunctions or disorders of nails and/or nail growth.

Said combination therapy is especially useful for prophylaxis and/or treatment of onychomycosis, onychocryptosis, nail psoriasis, melanonychia striata, white line disease, eczema, chronic onychia, discolored nails, thickened nails, and onychodystrophy.

As used herein, the term "plaster" refers to any device which can be applied to the nail and which comprises a contact layer which is pressed against the nail surface. Suitable plaster devices include plasters or preformed films based upon rubbers, acrylics, urethanes, silicone materials, polyvinylalkylethers, gels, and impregnated microporous membranes. Said plaster device could also be combined with or incorporated or formed into shape of an artificial or fake nail in order to improve cosmetic appearance.

As used herein, the term "plaster" refers to a preparation of at least one antimycotic agent, optionally together with suitable additives and/or binders, in a plaster device.

Furthermore, the present invention describes the use of the plaster for the transdermal and/or transnail prophylaxis and/or treatment of a dysfunction or disorder of nails and/or nail growth by adhesively securing to the nail and optionally the surrounding skin of said nail the plaster comprising a layer being designed to be in close contact with the nail and optionally with the surrounding skin. Said layer comprises:
a) an adhesive;
b) at least one skin and/or nail permeation enhancer;
c) at least one therapeutically effective amount of an antimycotic agent contained in said contact layer; and
d) at least one additive selected from the group comprising binders, cross linkers, softeners, solvents, fillers, and/or antioxidants.
Instead of the above-mentioned plaster the embodiment comprising two or more layer can also be used in order prevent and/or a dysfunction or disorder of nails and/or nail growth by adhesively securing to the nail and optionally the surrounding skin of said nail the plaster comprising:
a) a backing layer; and
b) a contact layer attached to said backing layer and being designed to be in close contact with the nail and optionally with the surrounding skin;
   said contact layer comprising:
   aa) an adhesive;
   bb) at least one skin and/or nail permeation enhancer;
   cc) at least one therapeutically effective amount of an antimycotic agent contained in said contact layer; and
   dd) at least one additive selected from the group comprising binders, cross linkers, softeners, solvents, fillers, and/or antioxidants.

One important aspect of the present invention is that the use of the plasters does not require the procedure of drilling at least one hole into the nail and/or daily scraping of the nail. Another advantage of the present invention is that the plasters are easy to use, convenient and user-friendly.

Furthermore, the inventive plaster can be used in combination with a systemic treatment of a dysfunction or disorder of nails and/or nail growth, such as onychomycosis, onychocryptosis, nail psoriasis, melanonychia striata, white line disease, eczema, chronic onychia, discolored nails, thickened nails, and onychodystrophy.

The present invention discloses a method for the transdermal and/or transnail delivery of a sufficient amount of at least one antimycotic agent to an affected nail, nail bed and surrounding tissue by adhesively securing to the nail and optionally the surrounding skin the plaster, in order to treat a dysfunction or disorder of growth of said nail.

One advantage of said inventive method is exhibited by the fact that this method does not require drilling holes into the nails and/or daily scraping of the nails.

The inventive method can also be applied in combination with a systemic treatment of onychomycosis or other systemic treatments for dysfunctions or disorders of nails and/or nail growth. Especially, a combination of the inventive method with a systemic treatment of onychomycosis or other systemic treatments for dysfunctions or disorders has been proven effective.

### Examples

The following examples shall exemplify the present invention and shall not limit the scope of the present invention to these specific embodiments.

The plasters or nail patches may preferably comprise a backing layer and/or a release liner. The backing layer, if present, is preferably made from PVC such as Guttagena PVC NBR foil and the release liner is preferably made of PET such as PET foil with both sides siliconized (100 µm).

### Example 1

### Plaster 1: Compounds of the contacting layer for 1.0 cm² plaster

| No. | Compound | Concentration |
|---|---|---|
| 1 | Sertaconazole | 1.4 mg |
| 2 | durotak 387-2825 | 8.80 mg |
| 3 | lactic acid | 0.11 mg |
| 4 | aerosil 200 | 0.33 mg |
| 5 | aluminum acetylacetonate | 0.11 mg |

The efficacy of the plaster was investigated in patients suffering from onychodystrophy of the fingernails.

One patient had a 40% dystrophic nail involvement at the start of treatment, which could be decreased to 15% after 24 weeks of treatment with the plaster. Another patient was treated for 24 weeks and the dystrophic nail area could be decreased from 20% to 5%. Another patient was also treated for 24 weeks with the plaster and the affected nail area was decreased from 30% to 10%.

Few treated patients showed only minor side effects during the treatment period. Said side effects were characterized as skin scaling of the tissue surrounding the nails.

One advantageous effect of the plasters disclosed herein is that the plaster has only to be replaced every week and not, for instance, daily. Therefore, an excess of the antimycotic agent is used in order to ensure that a sufficient amount of the antimycotic agent will after one week still be present in the adhesive layer of the plaster. The remaining amount of the antimycotic agent in the plaster has a prophylactic effect and prevents the development of infections and diminishes the risk of third persons to be infected by the patient.

The plasters 2 - 6 according to examples 2 - 6 give similar results.

The treatment period can last in isolated cases one year or longer. Normally, the treatment period will be one to several months under the condition that the plaster is replaced weekly.

### Example 2:

### Plaster 2: Composition for the manufacture of 1 cm² plaster

| No. | Compound | Amount |
|---|---|---|
| 1 | Clotrimazole | 1-2mg |
| 2 | Durotak 87-2852 solution (36.1 %) | 22.2 mg |
| 3 | Ethyl alcohol (96 %) | 2 mg |

The compound will be weighed and stirred until homogeneity. The mixture will be applied to a siliconized polyester sheet (thickness 75 µm, from Loparex, Apeldom, NL). The wet thickness of the glue film amounts 400 µm. Following 15 minutes drying at 60°C in drying cabinet and storage at 25°C for 12 hours the glue layer will be covered with polyolefine film of 50 µm thickness (Cotran No. 9722, from 3M-Medica, Borken, Germany).

Finally the self-adhesive plaster in the size of finger or toe nails will be punched from the sheet.

### Example 3:

### Plaster 3: Composition for the manufacture of 1 cm² plaster

| No. | Compound | Amount |
|---|---|---|
| 1 | Ciclopirox | 1.8 mg |
| 2 | Durotak 36-6172 solution (57.1 %) | 14 mg |
| 3 | n-Heptan | 2 mg |

The compound will be weighed and stirred until homogeneity. The mixture will be applied to a siliconized polyester sheet (thickness 75 µm, from Loparex, Apeldom, NL). The wet thickness of the glue film amounts 400 µm. Following 15 minutes drying at 60°C in drying cabinet and storage at 25°C for 12 hours the glue layer will be covered with polyolefine film of 50 µm thickness (Cotran No. 9722, from 3M-Medica, Borken, Germany).

Finally the self-adhesive plaster in the size of finger or toe nails will be punched from the sheet.

### Example 4:

### Plaster 4: Composition for the manufacture of 1 cm² plaster

| No. | Compound | Amount |
|---|---|---|
| 1 | Terbinafine | 1.5 mg |
| 2 | Durotak 87-2100 (52.9 %) | 15.1 mg |
| 3 | Ethyl alcohol (96 %) | 3 mg |

The compound will be weighed and stirred until homogeneity. The mixture will be applied to a siliconized polyester sheet (thickness 75 µm, from Loparex, Apeldom, NL). The wet thickness of the glue film amounts 400 µm. Following 15 minutes drying at 70°C in drying cabinet and storage at 25°C for 12 hours the glue layer will be covered with polyolefine film of 50 µm thickness (Cotran No. 9722, from 3M-Medica, Borken, Germany).

Finally the self-adhesive plaster in the size of finger or toe nails will be punched from the sheet.

### Example 5:

### Plaster 5: Composition for the manufacture of 1 cm² plaster

| No: | Compound | Amount |
|---|---|---|
| 1 | Amorolfine | 1.7 mg |
| 2 | Durotak 387-2516 solution (42.5 %) | 18.8 mg |
| 3 | Ethyl alcohol (96 %) | 3 mg |

The compound will be weighed and stirred until homogeneity. The mixture will be applied to a siliconized polyester sheet (thickness 75 µm, from Loparex, Apeldom, NL). The wet thickness of the glue film amounts 400 µm. Following 15 minutes drying at 60°C and 10 minutes at 80°C in drying cabinet and storage after cooling the glue layer will be covered with polyolefine film of 50 µm thickness (Cotran No. 9722, from 3M-Medica, Borken, Germany).

Finally the self-adhesive plaster in the size of finger or toe nails will be punched from the sheet.

### Example 6:

### Plaster 6: Compounds of the contacting layer for 1.0 cm² plaster

| No. | Compound | Concentration |
|---|---|---|
| 1 | EUDRAGIT^{®} E 100 | 42.2 g |
| 2 | Fluconazole | 17.7 g |
| 3 | dibutylsebacate | 19.0 g |
| 4 | succinic acid | 3.8 g |
| 5 | acetone | 21.0 g |
| 6 | isopropanol | 2.3 g |
| 7 | ethanol | 11.7 g |

### Equipment:

The solution is prepared in a high-speed stirred tank. The stirrer may be a dissolver disc, for example, which guarantees thorough mixing also at rising viscosity. On a laboratory scale, coating and drying are performed in a laboratory coating unit with integrated dryer (LTSV/LTF by W. Mathis AG, Switzerland).

### Instructions for processing:

Acetone, isopropanol, and ethanol is placed in a stirred tank and EUDRAGIT^{®} E 100 is added in portions over a period of 90 minutes. The stirrer is set to a speed which excludes sediment formation while dissolving EUDRAGIT^{®} E 100. Dibutylsebacate is added swiftly and stirring is continued for another 20 minutes. Thereafter fluconazole is added and succinic acid is given to the polymer solution in portions with intensive stirring. After complete addition of succinic acid the polymer solution is stirred for additional 20 minutes.

Coating is performed with the final polymer solution at the following parameters:

| | |
|---|---|
| Coating: | approximately 100 g of said polymer solution is applied to the backing layer foil (15 µm thickness, Revatrans^{®} MN, Tricon GmbH Freiburg) by means of a rotary doctor blade at a nip of 200 µm. |
| Drying: | Drying is performed at 60°C for 10 minutes, circulating air: 1500 m³/h, exhaust air: 80 m³/h. |

## Claims

1. Use of a plaster comprising a layer being designed to be in close contact with the nail and optionally with the surrounding skin;
said layer comprising:
a) an adhesive;
b) at least one skin and/or nail permeation enhancer;
c) at least one therapeutically effective amount of an antimycotic agent contained in said contact layer; and
d) at least one additive selected from the group comprising binders, cross linkers, softeners, solvents, fillers, and/or antioxidants,
for the transdermal and/or transnail prophylaxis and/or treatment of onychodystrophy, except onychomycosis.

2. Use of a plaster for the transdermal and/or transnail prophylaxis and/or treatment of onychodystrophy, except onychomycosis, by adhesively securing to the nail and optionally the surrounding skin of said nail the plaster comprising a layer being designed to be in close contact with the nail and optionally with the surrounding skin;
said layer comprising:
a) an adhesive;
b) at least one skin and/or nail permeation enhancer;
c) at least one therapeutically effective amount of an antimycotic agent contained in said contact layer; and
d) at least one additive selected from the group comprising binders, cross linkers, softeners, solvents, fillers, and/or antioxidants.

3. Use of a plaster comprising:
a) a backing layer; and
b) a contact layer attached to said backing layer and being designed to be in close contact with the nail and optionally with the surrounding skin;
said contact layer comprising:
aa) an adhesive;
bb) at least one skin and/or nail permeation enhancer;
cc) at least one therapeutically effective amount of an antimycotic agent contained in said contact layer; and
dd) at least one additive selected from the group comprising binders, cross linkers, softeners, solvents, fillers, and/or antioxidants,
for the transdermal and/or transnail prophylaxis and/or treatment of onychodystrophy, except onychomycosis.

4. Use of a plaster for the transdermal and/or transnail prophylaxis and/or treatment of onychodystrophy, except onychomycosis, by adhesively securing to the nail and optionally the surrounding skin of said nail the plaster comprising:
a) a backing layer; and
b) a contact layer attached to said backing layer and being designed to be in close contact with the nail and optionally with the surrounding skin;
said contact layer comprising:
aa) an adhesive;
bb) at least one skin and/or nail permeation enhancer;
cc) at least one therapeutically effective amount of an antimycotic contained in said contact layer; and
dd) at least one additive selected from the group comprising binders, cross linkers, softeners, solvents, fillers, and/or antioxidants.

5. Use according to claim 3 or 4, wherein said backing layer is an occlusive backing layer.

6. Use according to any one of claims 1 - 5, wherein the skin and/or nail permeation enhancer is selected from the group comprising fatty acids, fatty acid esters, fatty acid amides, fatty alcohols, 2-(2-ethoxyethoxy)-ethanol, esters of glycerol, glycerol monolaurate, propylene glycol, polyethylene glycols, unsaturated polyglycolized glycerides, saturated polyglycerides, a partial glyceride of ricinoleic acid, α-hydroxy acids, dimethylsulfoxide, decylmethylsulfoxide, pyrrolidones, salicylic acid, lactic acid, myristol, isopropyl myristate, dimethylformamide, dimethylacetamide, sodium dodecylsulfate, phospholipides, and proteolytic enzymes.

7. Use according to any one of claims 1 - 6 wherein the skin and/or nail permeation enhancer is contained in the contact layer in an amount of between 0.7% to 6% by weight of the contact layer.

8. Use according to any one of claims 1 - 7 wherein the additives are contained in the contact layer in an amount of between 15% to 20% by weight of the contact layer.

9. Use according to any one of claims 1 - 8 wherein said antimycotic agent is selected from the group comprising fluconazole (Diflucan^{®}), butoconazole, enilconazole, fenticonazole, sulconazole, naftifidine, clioquinol, iodoquinol, rimoprogin, griseofulvin, terbinafine (Lamisil^{®}), clotrimazole, itraconazole (Sempera^{®}), tioconazole, miconazole, tolnaftate, pyrogallol, econazole, isoconazole, terconazole, oxiconazole, voriconazole, amphotericin B, nystatin, tolciclate, sulbentine, ketoconazole, ciclopirox (Batrafen^{®}), amorolfine, bifonazole, sodium pyrithione, salicylic acid and/or salts of these agents.

10. Use according to any one of claims 1 - 9 wherein said antimycotic agent is contained in the adhesive in an amount of between 0.01 - 5 mg per cm² plaster.

11. Use according to any one of claims 1 - 10 wherein the adhesive is selected from the group comprising acrylic adhesives, rubber adhesives, and silicone adhesives.

12. Use according to any one of claims 1 - 11 wherein the adhesive is contained in the plaster in an amount of between 80% to 90% by weight of the contact layer.

13. Use according to any one of claims 1 - 12 in combination with a systemic treatment of onychocryptosis, nail psoriasis, melanonychia striata, white line disease, neurodermitis, eczema, chronic onychia, discolored nails, thickened nails, cleaved and split nails, Unguis inflexus, coilonychia, scleronychia, onychogryphosis, onychauxis, onychoschisis, onychorrhexis, trachynoychia, and other forms of onychodystrophy, except onychomycosis.

## Patentansprüche

1. Verwendung eines Pflasters, das eine Schicht umfasst, die so ausgestaltet ist, um in engem Kontakt mit dem Nagel und optional mit der umliegenden Haut zu sein;
wobei diese Schicht folgendes umfasst:
a) einen Klebstoff;
b) zumindest einen Haut- und/ oder Nagel-Permeationsförderer;
c) zumindest eine therapeutisch wirksame Menge eines in dieser Kontaktschicht enthaltenen Antimykotikums; und
d) zumindest ein Additiv ausgewählt aus der Gruppe umfassend Bindemittel, Quervernetzer, Weichmacher, Lösungsmittel, Füllmittel und/ oder Antioxidationsmittel,
für die transdermale und/ oder transunguale Prophylaxe und/ oder Behandlung von Onychodystrophie, ausgenommen Onychomykose.

2. Verwendung eines Pflasters für die transdermale und/ oder transunguale Prophylaxe und/ oder Behandlung von Onychodystrophie, ausgenommen Onychomykose, durch Aufkleben des Pflasters auf den Nagel und optional auf der umliegenden Haut dieses Nagels, wobei das Pflaster eine Schicht umfasst, die so ausgestaltet ist, um in engem Kontakt mit dem Nagel und optional mit der umliegenden Haut zu sein;
wobei diese Schicht folgendes umfasst:
a) einen Klebstoff;
b) zumindest einen Haut- und/ oder Nagel-Permeationsförderer;
c) zumindest eine therapeutisch wirksame Menge eines in dieser Kontaktschicht enthaltenen Antimykotikums; und
d) zumindest ein Additiv ausgewählt aus der Gruppe umfassend Bindemittel, Quervernetzer, Weichmacher, Lösungsmittel, Füllmittel und/ oder Antioxidationsmittel.

3. Verwendung eines Pflasters, das folgendes umfasst:
a) eine Rückschicht; und
b) eine an dieser Rückschicht angebrachte Kontaktschicht und die so ausgestaltet ist, um in engem Kontakt mit dem Nagel und optional mit der umliegenden Haut zu sein;
wobei diese Kontaktschicht folgendes umfasst:
aa) einen Klebstoff;
bb) zumindest einen Haut- und/ oder Nagel-Permeationsförderer;
cc) zumindest eine therapeutisch wirksame Menge eines in dieser Kontaktschicht enthaltenen Antimykotikums; und
dd) zumindest ein Additiv ausgewählt aus der Gruppe umfassend Bindemittel, Quervernetzer, Weichmacher, Lösungsmittel, Füllmittel und/ oder Antioxidationsmittel,
für die transdermale und/ oder transunguale Prophylaxe und/ oder Behandlung von Onychodystrophie, ausgenommen Onychomykose.

4. Verwendung eines Pflasters für die transdermale und/ oder transunguale Prophylaxe und/ oder Behandlung von Onychodystrophie, ausgenommen Onychomykose, durch Aufkleben des Pflasters auf den Nagel und optional auf der umliegenden Haut dieses Nagels, wobei das Pflaster folgendes umfasst:
a) eine Rückschicht; und
b) eine an dieser Rückschicht angebrachte Kontaktschicht und die so ausgestaltet ist, um in engem Kontakt mit dem Nagel und optional mit der umliegenden Haut zu sein;
wobei diese Kontaktschicht folgendes umfasst:
aa) einen Klebstoff;
bb) zumindest einen Haut- und/ oder Nagel-Permeationsförderer;
cc) zumindest eine therapeutisch wirksame Menge eines in dieser Kontaktschicht enthaltenen Antimykotikums; und
dd) zumindest ein Additiv ausgewählt aus der Gruppe umfassend Bindemittel, Quervernetzer, Weichmacher, Lösungsmittel, Füllmittel und/ oder Antioxidationsmittel.

5. Verwendung gemäß Anspruch 3 oder 4, wobei diese Rückschicht eine okklusive Rückschicht ist.

6. Verwendung gemäß einem der Ansprüche 1 bis 5, wobei der Haut- und/ oder Nagel-Permeationsförderer ausgewählt wird aus der Gruppe umfassend Fettsäuren, Fettsäureester, Fettsäureamide, Fettalkohole, 2-(2-Ethoxyethoxy)-Ethanol, Glycerolester, Glycerolmonolaurat, Propylenglykol, Polyethylenglykole, ungesättigte polyglykolisierte Glyceride, gesättigte Polyglyceride, ein Partialglycerid der Ricinolsäure, α-Hydroxysäuren, Dimethylsulfoxid, Decylmethylsulfoxide, Pyrrolidone, Salicylsäure, Milchsäure, Myristol, Isopropylmyristat, Dimethylformamid, Dimethylacetamid, Natriumdodecylsulfat, Phospholipide und proteolitische Enzyme.

7. Verwendung gemäß einem der Ansprüche 1 bis 6, wobei der Haut- und/ oder Nagel-Permeationsförderer in der Kontaktschicht in einer Menge von zwischen 0,7 und 6 Gewichtsprozent der Kontaktschicht enthalten sind/ist.

8. Verwendung gemäß einem der Ansprüche 1 bis 7, wobei die Additive in der Kontaktschicht in einer Menge von zwischen 15 und 20 Gewichtsprozent der Kontaktschicht enthalten sind.

9. Verwendung gemäß einem der Ansprüche 1 bis 8, wobei dieses Antimykotikum ausgewählt wird aus der Gruppe umfassend Fluconazol (Diflucan^{®}), Butoconazol, Enilconazol, Fentikonazol, Sulconazol, Naftifin, Clioquinol, lodoquinol, Rimoprogin, Griseofulvin, Terbinafin (Lamisil^{®}), Clotrimazol, Itraconazol (Sempera^{®}), Tioconazol, Miconazol, Tolnaftat, Pyrogallol, Econazol, Isoconazol, Terconazol, Oxiconazol, Voriconazol, Amphotericin B, Nystatin, Tolciclat, Sulbentin, Ketoconazol, Ciclopirox (Batrafen^{®}), Amorolfin, Bifonazol, Natriumpyrithion, Salicylsäure und/ oder Salze dieser Mittel.

10. Verwendung gemäß einem der Ansprüche 1 bis 9, wobei dieses Antimykotikum in dem Klebstoff in einer Menge von zwischen 0,01 und 5 mg pro cm² Pflaster enthalten ist.

11. Verwendung gemäß einem der Ansprüche 1 bis 10, wobei der Klebstoff ausgewählt wird aus der Gruppe umfassend Acrylklebstoffe, Gummiklebstoffe und Silikonklebstoffe.

12. Verwendung gemäß einer der Ansprüche 1 bis 11, wobei der Klebstoff in dem Pflaster in einer Menge von zwischen 80 und 90 Gewichtsprozent der Kontaktschicht enthalten ist.

13. Verwendung gemäß einem der Ansprüche 1 bis 12 in Kombination mit einer systemischen Behandlung von Onychocryptosis, Nagelpsoriasis, Melanonychia striata, der White-Line-Disease, Neurodermitis, Ekzemen, chronischer Onychie, verfärbten Nägeln, verdickten Nägeln, gespaltenen und gebrochenen Nägeln, Unguis inflexus, Koilonychie, Skleronychie, Onychogryphose, Onychauxis, Onychoschisis, Onychorrhexis, Trachyonychie und weiteren Formen von Onychodystrophie, ausgenommen Onychomykose.

## Revendications

1. Utilisation d'un pansement comprenant une couche destinée à être en contact étroit avec l'ongle et éventuellement avec la peau qui l'entoure;
ladite couche comprenant:
a) un adhésif;
b) au moins un amplificateur de perméation de la peau et/ ou de l'ongle; et
c) au moins une quantité efficace dans le cadre thérapeutique d'un agent antimycosique contenu dans ladite couche de contact; et
d) au moins un additif choisi parmi le groupe comprenant des liants, cross linkers, assouplisseurs, solvants, fillers et/ ou antioxydants,
pour la prophylaxie et/ ou le traitement transdermal(e)/transunguéal(e) et/ ou transdermaux/transunguéaux de l'onychodystrophie, sauf l'onychomycose.

2. Utilisation d'un pansement destinée à la prophylaxie et/ ou au traitement transdermal(e)/transunguéal(e) et/ou transdermaux/transunguéaux de l'onychodystrophie, sauf l'onychomycose, par l'application adhésive sur l'ongle et éventuellement sur la peau qui entoure ledit ongle du pansement comprenant une couche destinées à être en contact étroit avec l'ongle et éventuellement avec la peau qui l'entoure ;
ladite couche comprenant:
a) un adhésif;
b) au moins un amplificateur de perméation de la peau et/ ou de l'ongle; et
c) au moins une quantité efficace dans le cadre thérapeutique d'un agent antimycosique contenu dans ladite couche de contact; et
d) au moins un additif choisi parmi le groupe comprenant des liants, cross linkers, assouplisseurs, solvants, fillers et/ ou antioxydants.

3. Utilisation d'un pansement comprenant :
a) une couche de soutien ; et
b) une couche de contact attachée à ladite couche de soutien et destinée à être en contact étroit avec l'ongle et éventuellement avec la peau qui l'entoure;
ladite couche de contact comprenant:
aa) un adhésif;
bb) au moins un amplificateur de perméation de la peau et/ ou de l'ongle; et
cc) au moins une quantité efficace dans le cadre thérapeutique d'un agent antimycosique contenu dans ladite couche de contact; et
dd) au moins un additif choisi parmi le groupe comprenant des liants, cross linkers, assouplisseurs, solvants, fillers et/ ou antioxydants,
pour la prophylaxie et/ ou le traitement transdermal(e)/transunguéal(e) et/ ou transdermaux/transunguéaux de l'onychodystrophie, sauf l'onychomycose.

4. Utilisation d'un pansement destinée à la prophylaxie et/ ou au traitement transdermal(e)/transunguéal(e) et/ ou transdermaux/transunguéaux de l'onychodystrophie, sauf l'onychomycose, par l'application adhésive sur l'ongle et éventuellement sur la peau qui entoure ledit ongle du pansement comprenant:
a) une couche de soutien ; et
b) une couche de contact attachée à ladite couche de soutien et destinée à être en contact étroit avec l'ongle et éventuellement avec la peau qui l'entoure;
ladite couche de contact comprenant:
aa) un adhésif;
bb) au moins un amplificateur de perméation de la peau et/ ou de l'ongle; et
cc) au moins une quantité efficace dans le cadre thérapeutique d'un agent antimycosique contenu dans ladite couche de contact; et
dd) au moins un additif choisi parmi le groupe comprenant des liants, cross linkers, assouplisseurs, solvants, fillers et/ ou antioxydants.

5. Utilisation selon les revendications 3 ou 4, où ladite couche de soutien est une couche de soutien occlusive.

6. Utilisation selon l'une quelconque des revendications 1 à 5, où l'amplificateur de perméation de la peau ou de l'ongle est choisi parmi le groupe comprenant acides gras, esters d'acides gras, amides d'acides gras, alcools gras, 2-(2-éthoxyéthoxy)-éthanol, esters de glycérol, monolaurate de glycérol, propylèneglycol, polyéthylène glycols, glycérides polyglycolysées insaturées, polyglycérides saturées, une glycéride partielle de l'acide ricinoléique, α-hydroxyacide, diméthylsulfoxyde, sulfoxyde de décylméthyle, pyrrolidones, acide salicylique, acide lactique, myristol, myristate d'isopropyle, diméthylformamide, diméthylacétamide, docécylsulfate de sodium, phospolipides et enzymes protéolytiques.

7. Utilisation selon l'une quelconque des revendications 1 à 6, où la couche de contact contient une quantité d'amplificateur de perméation de la peau et/ ou de l'ongle d'entre 0,7 et 6 pourcents en poids de la couche de contact.

8. Utilisation selon l'une quelconque des revendications 1 à 7, où la couche de contact contient une quantité d'additifs d'entre 15 et 20 pourcents en poids de la couche de contact.

9. Utilisation selon l'une quelconque des revendications 1 à 8, où l'agent mycosique est choisi parmi le groupe comprenant Fluconazole (Diflucan^{®}), Butoconazole, Enilconazole, Fenticonazole, Sulconazole, Naftifine, Clioquinol, lodoquinol, Rimoprogrin, Griséofulvine, Terbinafine (Lamisil^{®}), Cotrimazol, Itraconazole (Sempera^{®}), Tioconazole, Miconazole, Tolnaftate, Pyrogallol, Econazole, Isoconazole, Terconazole, Oxiconazole, Voriconazole, Amphotéricine B, Nystatine, Tolciclate, Sulbentine, Kétoconazole, Ciclopirax (Batrafen^{®}), Amorolfine, Bifonazole, pyrithione de sodium, acide salicylique et/ ou des sels desdits agents.

10. Utilisation selon l'une quelconque des revendications 1 à 9, où l'adhésif contient une quantité dudit agent mycosique d'entre 0,01 et 5 mg par cm² du pansement.

11. Utilisation selon l'une quelconque des revendications 1 à 10, où l'adhésif est choisi parmi le groupe comprenant des adhésifs acryliques, des adhésifs en caoutchouc et des adhésifs siliconés.

12. Utilisation selon l'une quelconque des revendications 1 à 11, où le pansement contient une quantité d'adhésif d'entre 80 et 90 pourcents en poids de la couche de contact.

13. Utilisation selon l'une quelconque des revendications 1 à 12 en combinaison avec un traitement systémique de l'onychocryptose, du psoriasis des ongles, de la mélanonychie longitudinale, de la maladie nommée White-Line-Disease, de la neurodermite, des eczémas, de l'onychie chronique, des ongles décolorés, des ongles épaissis, des fissures au niveau des ongles et des ongles cassés, de l'unguis inflexus, de la koïlonychie, de la scleronychie, de l'onychogryphose, de l'onychauxis, de l'onychoschizie, de l'onychorrhexis, de la trachyonychie et d'autres formes de l'onychodystrophie, sauf l'onchychomycose.
